# EUROPEAN PATENT SPECIFICATION

(11) **EP 4 618 097 B1**
(45) Date of publication and mention of the grant of the patent: **18.11.2026**
(21) Application number: 25192472.6
(22) Date of filing: 04.07.2023
(51) Int. Cl.: A61B 34/20, G16H 40/40

(54) **TECHNIQUE FOR SUPPORTING USERS IN AN OPERATING ROOM BY TRIGGERING FEEDBACK REGARDING MEDICAL INSTRUMENTS**
TECHNIK ZUR UNTERSTÜTZUNG VON BENUTZERN IN EINEM OPERATIONSSAAL DURCH AUSLÖSUNG VON FEEDBACK BEZÜGLICH MEDIZINISCHER INSTRUMENTE
TECHNIQUE PERMETTANT DE PRENDRE EN CHARGE DES UTILISATEURS DANS UNE SALLE D'OPÉRATION PAR DÉCLENCHEMENT D'UNE RÉTROACTION CONCERNANT DES INSTRUMENTS MÉDICAUX

(43) Date of publication of application: 17.09.2025
(62) Document number(s) of the earlier application(s) in accordance with Art. 76 EPC: 23183394.8 / 4 487 804
(73) Proprietor: Stryker European Operations Limited, Carrigtwohill, Co. Cork T45 HX08 (IE)
(72) Inventor: KHORWAL, Renu, 110063 New Delhi (IN); DUBE, Saurabh Matacharan, 400002 Mumbai (IN); RAINA, Ravi Kiran, 201301 Noida (IN); BEDI, Inderjeet Singh, 110032 Delhi (IN); WEEDE, Oliver, 79110 Freiburg (DE); MOCTEZUMA de la BARRERA, José Luis, 79104 Freiburg (DE)
(74) Representative: Schott, Jakob Valentin

(56) References cited:
- WO-A1-2023/002382
- US-A1- 2019 228 859

## Description

### Technical Field

The present disclosure generally relates to a method for supporting users in an operating room by triggering feedback regarding one or more medical instruments. A related system, computer program and carrier are also disclosed herein.

### Background

In many clinical scenarios, clinical personnel such as surgeons wish to be provided with feedback regarding tracked poses of medical instruments. For example, surgeons may wish to be informed on a current pose of a handheld drill relative to a patient's body, or on an alignment of a pedicle screw driver in relation to a medical tool, such as a trocar, handled by a robot.

Some surgical navigation systems can provide a surgeon with a single navigation view visualizing a tracked pose of a handheld medical instrument relative to a patient's body. In case multiple medical instruments are used, tracked poses of the multiple medical instruments may be visualized at the same time in the single navigation view. Especially if the tracked medical instruments are located far apart from one another (e.g., at different vertebral levels of a patient's spine), or if multiple medical instruments are handled simultaneously by different surgeons, a single navigation view may not be optimal for navigating each of the medical instruments.

Some surgical navigation systems enable a user to select one of a plurality of tracked medical instruments and subsequently generate a single navigation view tailored to the selected medical instrument. This approach requires a user interaction with the navigation system and only provides feedback for the selected medical instrument.

WO 2023/002382 A1 describes systems, methods, and/or instrumentalities for a surgical hub configuring a display may be provided. In examples, a health care provider and/or a medical instrument may be tracked within an operating room.

US 2019/228859 A1 discloses a surgical workflow system for performing a surgical procedure. The surgical workflow system comprises one or more locators arrangeable to determine locations of a plurality of surgical objects. Information conveyor devices are coupled to the one or more locators and are assigned to different participants in the surgical procedure. A workflow controller accesses a pre-scripted workflow having a plurality of workflow steps associated with the surgical procedure, identifies a change event that indicates a deviation from the pre-scripted workflow, determines event information tailored to the different participants based on the change event, and transmits the event information to the participants through the plurality of information conveyor devices so that different event information can be conveyed to the different participants, wherein the event information is provided in the context of the surgical objects.

### Summary

There is a need for a technique for supporting users in an operating room that solves one or more of the aforementioned or other problems.

The present invention is defined by the scope of each of independent claims 1, 13 and 15. Specific embodiments within said scope are provided in the remaining dependent claims. All subsequently described aspects are only according to the present invention if they are within the scope of at least one of the independent claims 1, 13 and 15.

According to a first aspect, a method for supporting users in an operating room by triggering feedback regarding one or more medical instruments is provided. The method is performed by at least one processor. The method comprises obtaining spatial information indicative of a plurality of spatial regions in the operating room, each of the plurality of spatial regions being associated with one or more feedback parameters. The method further comprises obtaining tracking information indicative of tracked poses of a plurality of medical instruments in the operating room. The method comprises associating, based on the spatial information and the tracking information, each of the plurality of medical instruments to a respective at least one of the plurality of spatial regions. The method comprises triggering feedback, for each of the plurality of medical instruments, according to the one or more feedback parameters of the associated respective at least one of the plurality of spatial regions.

The one or more feedback parameters may be region-specific. The one or more feedback parameters may differ between two or more (e.g., all) of the plurality of spatial regions.

The plurality of spatial regions may be defined based on a pose of at least one reference object in the operating room.

The at least one reference object may comprise at least a portion of a patient's body. The at least one reference object may comprise at least one anatomical element (e.g., a bone such as a vertebra) of the patient's body. The at least one reference object may comprise at least a portion of a medical tool, for example a distal tool tip. The at least one reference object may comprise at least a portion of a medical instrument, for example a distal tip of the medical instrument.

In one example, different ones of the plurality of spatial regions comprise different portions of the patient's body.

At least some of the plurality of spatial regions may be separated from one another by one or more virtual planes. The virtual planes may be defined relative to the patient's body (e.g., relative to one or more anatomical elements of the patient's body).

The one or more virtual planes may comprise at least one anatomical plane, at least one user-defined plane and/or at least one plane associated with an anatomical element of the patient's body.

In one example, the medical tool is handled by a robot in the operating room.

At least one of the spatial information and the tracking information may be obtained for multiple points in time. The feedback may be iteratively triggered for two or more (e.g., each) of the multiple points in time.

One or more of the associated respective at least one of the plurality of spatial regions may be updated based on a movement of one or more of the plurality of medical instruments as indicated by the tracking information obtained for multiple points in time.

The associated respective at least one of the plurality of spatial regions may be updated such that the medical instrument associated with said respective at least one of the plurality of spatial regions remains within said at least one of the plurality of spatial regions. Alternatively, or in addition, the associated respective at least one of the plurality of spatial regions may be updated such that a medical instrument not associated with said respective at least one of the plurality of spatial regions remains outside said at least one of the plurality of spatial regions.

In one variant, the plurality of medical instruments comprises instruments handled simultaneously.

The instruments handled simultaneously may be handled by different surgeons.

In one example, different subsets of the plurality of spatial regions are associated with different surgeons.

The one or more feedback parameters may define at least one of an auditory feedback, a haptic feedback and a visual feedback.

The visual feedback may include display of a navigation view visualizing the pose of the respective medical instrument.

In one example, the one or more feedback parameters define at least one setting of the navigation view, the at least one setting comprising: a type of medical image data used for rendering the navigation view; a criterion for highlighting structures in the navigation view; a criterion for indicating planned objects in the navigation view; an orientation of the navigation view; and/or a perspective of the navigation view.

According to a second aspect, a system is provided. The system comprises at least one processor configured to: obtain spatial information indicative of a plurality of spatial regions in the operating room, each of the plurality of spatial regions being associated with one or more feedback parameters; obtain tracking information indicative of tracked poses of a plurality of medical instruments in the operating room; associate, based on the spatial information and the tracking information, each of the plurality of medical instruments to a respective at least one of the plurality of spatial regions; and trigger feedback, for each of the plurality of medical instruments, according to the one or more feedback parameters of the associated respective at least one of the plurality of spatial regions. The at least one processor may be configured to perform the method according to the first aspect.

The system may further comprise a tracking system configured to track the poses of the plurality of medical instruments in the operating room. Alternatively, or in addition, the system may comprise a feedback unit configured to provide the feedback to at least one user in the operating room. The system may comprise a robot configured to handle a medical tool.

According to a third aspect, a computer program is provided. The computer program comprises instructions which, when executed on at least one processor, cause the at least one processor to: obtain spatial information indicative of a plurality of spatial regions in the operating room, each of the plurality of spatial regions being associated with one or more feedback parameters; obtain tracking information indicative of tracked poses of a plurality of medical instruments in the operating room; associate, based on the spatial information and the tracking information, each of the plurality of medical instruments to at least one of the plurality of spatial regions; and trigger feedback, for each of the plurality of medical instruments, according to the one or more feedback parameters of the associated at least one of the plurality of spatial regions. The computer program may comprise instructions which, when executed on the at least one processor, cause the at least one processor to perform the method according to the first aspect.

According to a fourth aspect, a carrier is provided. The carrier carries the computer program according to the third aspect. The carrier may carry a computer program comprising instructions which, when executed on the at least one processor, cause the at least one processor to perform the method according to the first aspect. For example, a carrier (e.g., a non-transitory computer storage medium) is provided carrying (e.g., storing) a computer program comprising instructions which, when executed on at least one processor, cause the at least one processor to: obtain spatial information indicative of a plurality of spatial regions in the operating room, each of the plurality of spatial regions being associated with one or more feedback parameters; obtain tracking information indicative of tracked poses of a plurality of medical instruments in the operating room; associate, based on the spatial information and the tracking information, each of the plurality of medical instruments to at least one of the plurality of spatial regions; and trigger feedback, for each of the plurality of medical instruments, according to the one or more feedback parameters of the associated at least one of the plurality of spatial regions.

### Brief Description of the Drawings

Further details, advantages and aspects of the present disclosure will become apparent from the following taken in conjunction with the drawings, wherein:
Fig. 1 shows a schematic illustration of a system in accordance with the present disclosure;
Fig. 2 shows a flowchart of a method in accordance with the present disclosure;
Figs. 3a-3f schematically illustrate spatial regions in accordance with the present disclosure; and
Figs. 4a-4f schematically illustrate different scenarios and corresponding spatial regions in accordance with the present disclosure.

### Detailed Description

In the following description, reference to the drawings will be made. The same reference numerals will be used to denote the same or similar structural features.

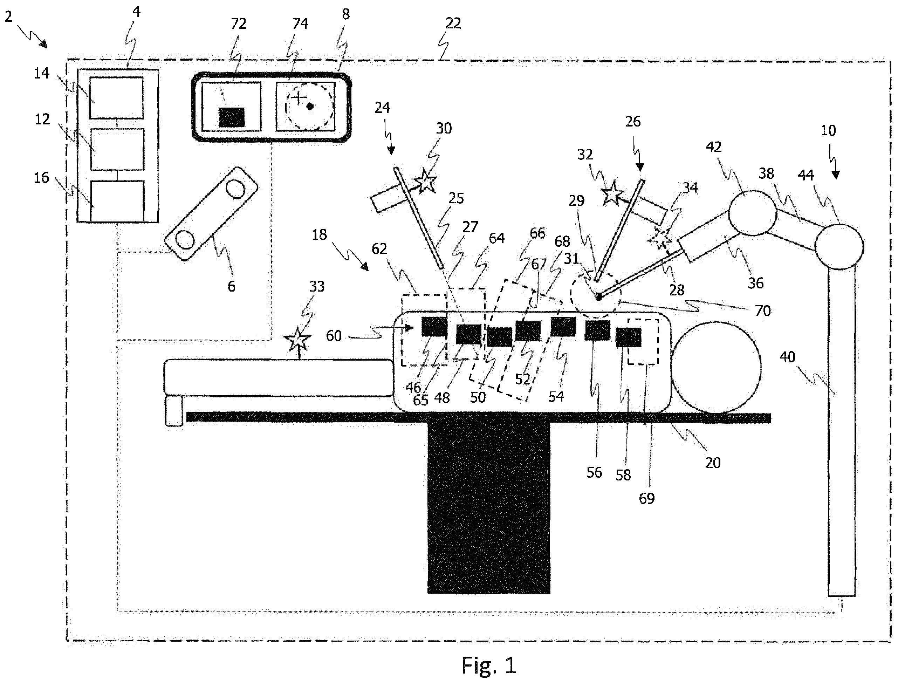

Fig. 1 shows a schematic illustration of a system 2 in accordance with the present disclosure. The system 2 may be referred to as a surgical navigation system and comprises a computing system 4. The system 2 may further comprise a tracking system 6, a display unit 8 and/or a robot 10. The computing system 4 comprises at least one processor 12 communicatively connected to at least one memory 14 and at least one interface 16. The at least one interface may be communicatively connected to the tracking system 6, the display unit 8 and/or the robot 10 via one or more wired or wireless connections.

Also shown is a patient's body 18 of a patient lying on a treatment bed 20 in an operating room 22, two medical instruments 24, 26 and a medical tool 28. Each of the medical instruments 24, 26 in this example is a handheld instrument such as a pointer, a drill, a chisel or a screwdriver. The medical instruments 24, 26 may be handled simultaneously, for example by different surgeons. The medical instrument 24 comprises a shaft 25 extending longitudinally along a shaft axis 27. The medical instrument 26 comprises a distal instrument tip 29. The medical tool 28 in the illustrated example is (e.g., simultaneously) handled by the robot 10 and comprises a distal tool tip 31.

The tracking system 6 is configured to track poses of the medical instruments 24, 26 by locating (e.g., optical or electromagnetic) trackers 30, 32 attached to the medical instruments 24, 26. The tracking system 6 may also track a pose of the medical tool 28 by locating a tracker 34 attached thereto. The tracking system 6 may be configured as an optical tracking system and may comprise a (e.g., stereo-) tracking camera for locating optical trackers. The tracking system 6 may alternatively be configured as an electromagnetic tracking system and may comprise an electromagnetic field generator.

Alternatively to tracking the medical tool 28 with the tracking system 6, a pose of the medical tool 28 may be determined (e.g., by the at least one processor 12) based on a pose of the robot 10, for example based on rotations and/or translations of actuators of the robot 10. In the illustrated example, a pose of the medical tool 28 may be determined based on angular orientations of arm segments 36, 38, 40 of the robot 10 defined by joints 42, 44 between the arm segments 36, 38, 40 of the robot 10.

The tracking system 6 may be further configured to track a pose of the patient's body 18 by locating a patient tracker 33 arranged in a fixed spatial relationship relative to the patient's body 18 (e.g., adhesively attached to the patient's skin or clamped to an anatomical element of the patient's body). A transformation, also known as a registration, between (e.g., pre-operative) patient image data of at least a portion of the patient's body 18 on the one hand and the patient's body 18 on the other hand may be used to transform (e.g., pre-planned) locations defined relative to the patient image data into (e.g., real-world) locations in the operating room 22. Various techniques for determining such a transformation or registration are known to those skilled in the art. For example, points acquired on a surface of the patient's body 18 using a tracked registration probe may be matched to a surface of the patient's body 18 as represented by the patient image data to obtain the transformation.

The display unit 8 is an example of a feedback unit configured to provide feedback to at least one user, in particular feedback regarding the medical instrument(s) 30, 32. Other examples of such a feedback unit include a haptic feedback unit (e.g., included in one or more of the instruments 30, 32), another type of visual feedback unit such as an indicator light (e.g., included in one or more of the instruments 30, 32), and an auditory feedback unit such as a speaker.

The patient's body 18 comprises a plurality of anatomical elements such as organs or bones. In the illustrated example, vertebrae 46-58 of the patient's spine 60 are indicated as examples of anatomical elements of the patient's body 18. It is noted that although a patient's spine 60 typically comprises a total of 33 vertebrae, only seven vertebrae 46-58 are illustrated in Fig. 1 to provide a better overview. Also indicated in Fig. 1 are a plurality of spatial regions 60-68.

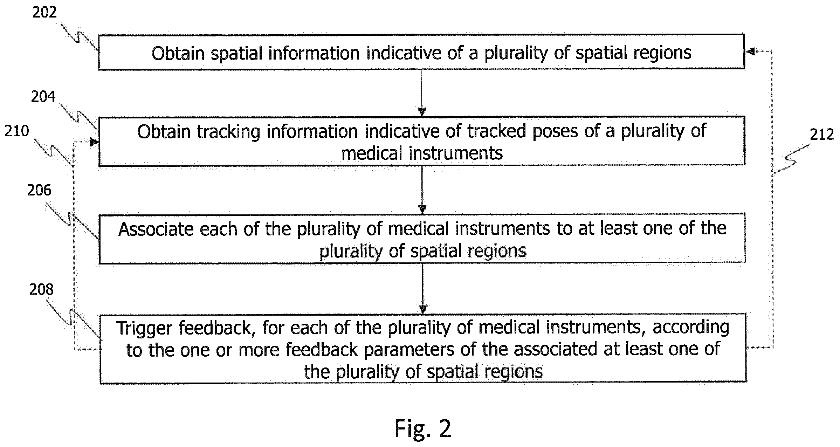

Fig. 2 shows a flowchart of a method in accordance with the present disclosure. The method may be performed by the at least one processor 12 of the system 2. To this end, the at least one memory 14 may store a computer program comprising instructions which, when executed by the at least one processor 12, cause the at least one processor to perform the method disclosed herein. The at least one memory 14 is an example of a carrier carrying the computer program. The computer program may alternatively be carried by a data stream received by the at least one processor 12 via the interface 16, for example from a server. Although reference signs of Fig. 1 will be used in the following, these are not to be understood as limiting the method to the specific details shown in Fig. 1.

The method may be referred to as a computer-implemented method. In one particular variant, the method is not a method for treatment of the human or animal body by surgery or therapy and/or the method does not comprise a surgical step.

In step 202, spatial information indicative of a plurality of spatial regions 62-70 in the operating room 22 is obtained.

The spatial information may indicate, for at least two or all of the spatial regions, at least one property selected from a size, a shape, an outline, a border, a position and an orientation. The spatial information may be obtained from the at least one memory 14. The spatial information may be predefined and/or pre-planned, in particular before a surgical procedure is started. The spatial information may be determined by the at least one processor 12 or defined by a user.

The spatial information may be determined or defined based on (e.g., pre-operative) patient image data comprising one or more medical images of at least a portion of the patient's body 18 such as computed tomography, CT, images and/or magnetic resonance, MR, images. The spatial information may be indicative of the spatial regions 62-70 in a real-world coordinate system, for example a coordinate system of the patient's body 18 and/or the patient tracker 33. The method may comprise obtaining (e.g., predefined and/or pre-planned) information indicative of the spatial regions 62-70 (e.g., defined based on or relative to one or more anatomical elements of the patient's body represented by the patient image data) in a coordinate system of the patient image data, obtaining a transformation between the coordinate system of the patient image data and a (e.g., the) real-world coordinate system, and determining the spatial information by transforming the information indicative of the spatial regions 62-70 from the coordinate system of the patient image data into the real-world coordinate system based on the obtained transformation. As explained above, various techniques exist for determining such a transformation, also referred to as registration between the patient image data and the patient's body 18.

One or more (e.g., all) of the plurality of spatial regions 62-70 may be defined (e.g., in the real-world coordinate system) based on a pose of at least one reference object in the operating room 22. For example, one or more (e.g., all) of the plurality of spatial regions 62-70 may be defined relative to the pose of the at least one reference object in the operating room 22 (e.g., in the real-world coordinate system). The at least one reference object may comprise at least a portion of the patient's body 18 (e.g., one or more anatomical elements of the patient's body 18 such as one of the vertebrae 46-58) and/or at least a portion of the medical tool 28 (e.g., the distal tip 31 of the medical tool 28) and/or at least a portion of a medical instrument 24, 26.

The plurality of spatial regions 62-70 may differ from one another in at least one property selected from a size, a shape, an outline, a border, a position and an orientation. The plurality of spatial regions 62-70 may differ from one another in one or more anatomical elements of the patient's body 18 comprised in the respective spatial region. In other words, different ones of the plurality of spatial regions 62-70 may comprise different portions of the patient's body (e.g., different anatomical elements such as different vertebrae 46-58). Each of the spatial regions 62-70 may be a three-dimensional region. Two or more (e.g., all) of the spatial regions 62-70 may be spatially disjunct. Two or more of the spatial regions 62-70 may overlap one another (e.g., at least partially).

At least some (e.g., two or more) of the plurality of spatial regions 62-70 may be separated from one another by one or more virtual planes 65, 37. The one or more virtual planes 65, 67 may be defined relative to the patient's body 18, for example relative to one or more anatomical elements (e.g., one or more vertebrae 46-58) of the patient's body 18. The one or more virtual planes 65, 67 may comprise at least one anatomical plane (e.g., a coronal plane, a sagittal plane or a transverse plane), at least one user-defined plane and/or at least one plane associated with an anatomical element of the patient's body 18 (e.g., one of the vertebrae 46-58). The method may comprise obtaining information (e.g., from a DICOM header of the patient image data) indicative of an orientation of the at least one virtual plane 65, 67 in the coordinate system of the patient image data and transforming the orientation into the real-world coordinate system, using the known registration between these two coordinate systems, to obtain the two or more spatial regions 62-70, which separated by the at least one virtual plane 65, 67, in the real-world coordinate system.

Different subsets of the plurality of spatial regions may be associated with different surgeons. That is, different spatial regions may be defined for different surgeons preoperatively. Each of the subsets of the plurality of spatial regions may correspond to a different side of the patient's spine 60, for example relative to an anatomical plane. The subsets may consist of subset-specific spatial regions. That is, none of the plurality of spatial regions may be associated with more than one surgeon.

Each of the plurality of spatial regions 62-70 is associated with one or more feedback parameters.

The one or more feedback parameters may be predefined and/or obtained from the at least one memory 14. The one or more feedback parameters may be defined by a user. The one or more feedback parameters may differ between two or more of the plurality of spatial regions. In one example, the one or more feedback parameters are region-specific. The one or more feedback parameters may be specific for anatomical elements of the patient's body comprised in the respective spatial regions. This may enable a feedback that is tailored specifically for the individual spatial zones.

In step 204, tracking information indicative of tracked poses of a plurality of medical instruments in the operating room is obtained (e.g., tracked poses of the instruments 24, 26 in the real-world coordinate system).

The tracking information may be obtained from the tracking system 6 by the at least one processor 12, for example via the at least one interface 16. Alternatively, the tracking information may be loaded from the at least one memory 14. The tracking information may be indicative of exactly one pose per medical instrument 24, 26, for example tracked at a same point in time. Thus, the tracking information may indicate poses of the tracked medical instruments 24, 26 relative to one another.

The order of steps 202 and 204 may be reversed compared with Fig. 2. In another variant, the spatial information and the tracking information are obtained simultaneously (e.g., as part of a single dataset) such that steps 202 and 204 are performed in unison.

In step 206, each of the plurality of medical instruments (e.g., 24, 26) is associated to a respective at least one of the plurality of spatial regions (e.g., 62-70), based on the spatial information and the tracking information.

Each of the medical instruments 24, 26 may be associated to a subset of the spatial regions 62-70, or to exactly one of the spatial regions 62-70. For example, a medical instrument may be associated to a spatial region 62-70 in case one or more predefined spatial constraints of this spatial region 62-40 are met. The one or more predefined spatial constraints may be obtained from the at least one memory 16. The method (e.g., step 206) may comprise obtaining the one or more predefined spatial constraints for one or more of the plurality of spatial regions 62-70 and determining, based on the spatial data and the tracking data, whether the one or more predefined spatial constraints are met. The one or more predefined spatial constraints may comprise at least one of (i) a maximum distance between (e.g., at least one part of) the instrument (e.g., the instrument's distal tip) and the spatial region, (ii) a maximum distance between (e.g., at least one part of) the instrument (e.g., the instrument's distal tip) and an anatomical element enclosed by and/or defining the spatial region, (iii) a maximum deviation of a main instrument axis (e.g., a longitudinal shaft axis 27) from the spatial region and (iv) a maximum deviation of a main instrument axis (e.g., a longitudinal shaft axis 27) from an anatomical element enclosed by and/or defining the spatial region. A spatial region closest to the medical instrument and/or a spatial region pointed at by the medical instrument may be associated with that medical instrument or vice versa.

In the example of Fig. 1, the medical instrument 24 may be associated to the spatial region 64, as the longitudinal shaft axis 27 intersects the spatial region 64 which means the predefined spatial constraint of a maximum deviation of the longitudinal shaft axis 27 from the spatial region 64 is met. The medical instrument 26 may be associated to the spatial region 70, as the distal tip 29 lies within the spatial region 70 which means the predefined spatial constraint of a maximum distance of the distal tip 29 from the spatial region 70 is met.

In one variant, predefined association information is obtained indicative of associations of each of the plurality of medical instruments (e.g., 24, 26) to a respective at least one of the plurality of spatial regions (e.g., 62-70). The associations may be defined by a user. The associations may be determined based on a priority associated with each of the plurality of medical instruments.

In step 208, for each of the plurality of medical instruments, feedback is triggered, according to the one or more feedback parameters of the associated respective at least one of the plurality of spatial regions.

The triggered feedback may be based on at least one of the tracking information and the spatial information. The feedback triggered for a medical instrument may be based on the tracked pose of this medical instrument (e.g., as indicated by the tracking information) and based on the associated (e.g., respective) at least one of the plurality of spatial regions. The feedback triggered for a medical instrument may be indicative of the tracked pose of this medical instrument, for example relative to the associated (e.g., respective) at least one of the plurality of spatial regions. The one or more feedback parameters associated with a spatial region may define how the tracked pose of the medical instrument associated with this spatial region is indicated by the feedback triggered for this medical instrument.

The one or more feedback parameters may define at least one of an auditory feedback (e.g., a warning tone), a haptic feedback (e.g., a vibration) and a visual feedback (e.g., display of an image on the display unit 8 or activation of a warning lamp). The visual feedback may include display of a navigation view 72, 74 visualizing the pose of the respective medical instrument 24, 26 as indicated by the tracking information. In this case, the one or more feedback parameters may define at least one setting of the navigation view 72, 74, the at least one setting comprising: a type of medical image data used for rendering the navigation view 72, 74; a criterion for highlighting structures in the navigation view 72, 74; a criterion for indicating planned objects in the navigation view 72, 74; an orientation of the navigation view 72, 74; and/or a perspective of the navigation view 72, 74.

In the example of Fig. 1, the navigation view 72 may indicate a pose of the medical instrument 24 by visualizing the longitudinal shaft axis 27 relative to the anatomical element encased by the spatial region 64, namely the vertebra 48. In this example, the one or more feedback parameters associated with the spatial region 64 may define a visual feedback including display of the navigation view 72, and may define a perspective of the navigation view as a lateral perspective. The navigation view 74 may indicate a pose of the medical instrument 26 by visualizing the location of the tip 29 in the form of crosshairs relative to the location of the tool tip 31, and by further visualizing the spatial region 70. In this example, the one or more feedback parameters associated with the spatial region 70 may define a visual feedback including display of the navigation view 74, and may define a perspective of the navigation view as a perspective along a main axis of the instrument's 26 shaft.

At least one of the spatial information and the tracking information may be obtained for multiple points in time and the feedback may be iteratively triggered for two or more (e.g., each) of the multiple points in time. The method may be at least partially repeated by updating at least one of the spatial information and the tracking information and performing the subsequent steps 206-208 using the updated information, as indicated in Fig. 2 with dashed arrows 210, 212. This may ensure that the navigation view 72, 74 indicates a current pose of a medical instrument 24, 26 and may thus be particularly advantageous for guiding a user.

One or more of the associated respective at least one of the plurality of spatial regions 62-70 may be updated based on a movement of one or more of the plurality of medical instruments 24, 26 as indicated by the tracking information obtained for multiple points in time. For example, the associated respective at least one of the plurality of spatial regions (e.g., 70) may be updated such that the medical instrument (e.g., 26) associated with said respective at least one of the plurality of spatial regions remains within said at least one of the plurality of spatial regions. Alternatively, or in addition, the associated respective at least one of the plurality of spatial regions (e.g., 70) may be updated such that a medical instrument (e.g., 24) not associated with said respective at least one of the plurality of spatial regions remains outside said at least one of the plurality of spatial regions. Thus, spatial zones may be updated based on a movement of the medical instruments 24, 26 associated or not associated with these spatial zones. This may ensure that the association between a spatial zone and a medical instrument is maintained over time.

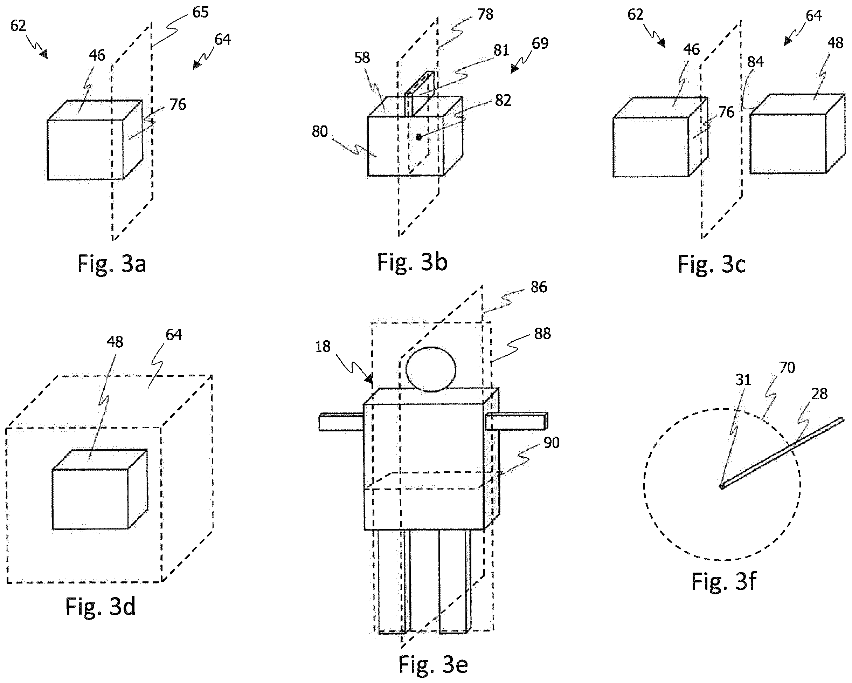

Figs. 3a-3f schematically illustrate examples of spatial regions in accordance with the present disclosure.

In the example of Fig. 3a, a virtual plane 65 defines a boundary between two adjacent spatial regions 62, 64. In this example, the virtual plane 65 is defined based on an anatomical element of the patient's body 18, namely a vertebra 46 of the patient's spine 60. A surface of the anatomical element, namely an endplate 76 of the vertebra 46, lies in the virtual plane 65.

In the example of Fig. 3b, a virtual plane 78 defines a border of a spatial region 69. In this example, the virtual plane 78 is defined relative to an anatomical element of the patient's body 18, namely a vertebra 58 of the patient's spine 60. The virtual plane 78 extends axially along a middle of the spinous process 81 of the vertebra 58. The virtual plane 81 may be perpendicular to the endplate 80 of the vertebra 58, and intersect a center 82 of the vertebra 58.

In the example of Fig. 3c, a virtual plane 65 defines a boundary between two adjacent spatial regions 62, 64. In this example, the virtual plane 65 is defined based on two anatomical elements of the patient's body 18, namely two vertebrae 46, 48 of the patient's spine 60. The virtual plane 65 extends (e.g. equidistantly) between the two anatomical elements and in the illustrated example is parallel to an endplate 76, 84 of each vertebra 46, 48.

In the example of Fig. 3d, a spatial region 64 is defined as a bounding box around an anatomical element of the patient's body 18, for example a vertebra 48. The bounding box may have a predefined shape such as a square or a sphere, or may define an area with a predefined thickness around the enclosed anatomical element, thereby representing an enlarged version of the anatomical element.

In the example of Fig. 3e, a plurality of virtual planes 86, 88, 90 are illustrated. The virtual planes 86, 88, 90 correspond to different anatomical planes of the patient's body 18. Each of the virtual planes 86, 88, 90 may be a border of one or more spatial regions and/or may separate adjacent regions from one another. In the example of Fig. 3e, a maximum of 8 spatial regions may be defined by the virtual planes 86, 88, 90.

In the example of Fig. 3f, a spherical spatial region 70 is illustrated, having a predefined radius (e.g., several cm) around a tool tip 31 of the medical tool 28.

Other variants of spatial regions are also possible. For instance, a spatial region may be defined by a user (e.g., in an arbitrary pose relative to the patient's body). One or more spatial regions may be determined based on a segmentation of at least one anatomical element encased by the one or more spatial regions, such as in the example of Fig. 3d.

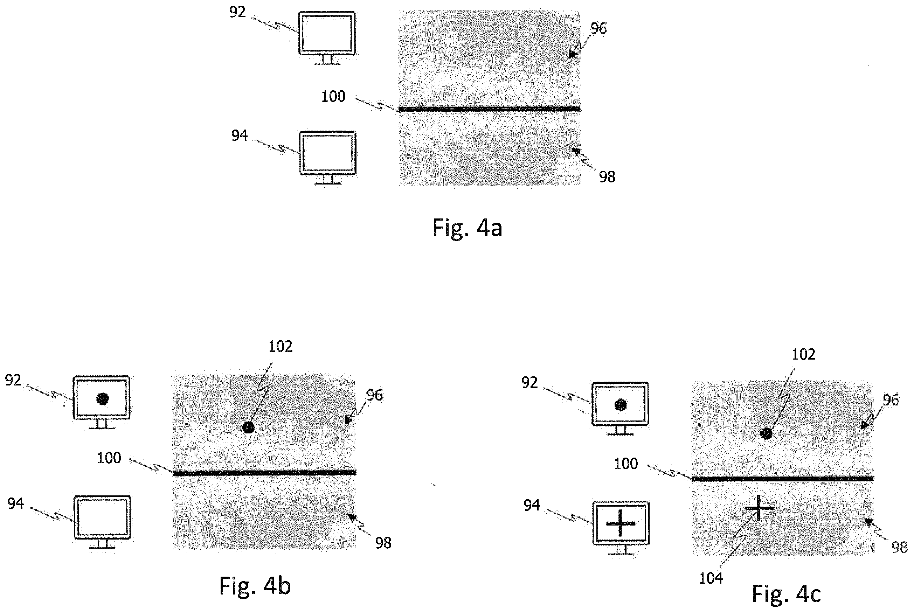

Figs. 4a-4f schematically illustrate different scenarios and corresponding spatial regions in accordance with the present disclosure.

Fig. 4a shows two navigation views 92, 94 that may be displayed on a same display unit 8 or on different display units 8. A medical image of the patient's body 18 is also shown in Fig. 4a, on which spatial regions 96, 98 are indicated that are separated from one another by a virtual plane 100. In this example, the virtual plane 100 defining the spatial regions 96, 98 may correspond to the sagittal plane of the patient's body 18. Thus, the virtual plane 100 is static and the spatial zones 96, 98 shown in Fig. 4a may be referred to as static or initial spatial zones. One surgeon may operate in the spatial zone 96 and another surgeon may operate in the spatial zone 98. As no tracked instruments are visible to the tracking system 6 in the scenario of Fig. 4a, no such instruments are visualized in the navigation views 92, 94.

In the scenario of Fig. 4b, a surgeon has positioned a tip of a medical instrument (e.g., 26) at a position 102 relative to the patient's body 18. The tracking system 6 allows determining the relative pose between that medical instrument and the spatial zones 96, 98. The tracked instrument is associated to the spatial zone 96 in which it is located, and a corresponding navigation view 92 is displayed, indicating the pose of that medical instrument relative to the patient's body 18.

In the scenario of Fig. 4c, another surgeon has positioned a tip of another medical instrument (e.g., 26) at a position 104 relative to the patient's body 18. The tracking system 6 allows determining the relative pose between that medical instrument and the spatial zones 96, 98. The instrument at position 104 is associated to the spatial zone 98 in which its tip is located, and a corresponding navigation view 94 is displayed, indicating the pose of that medical instrument relative to the patient's body 18. As can be seen in Fig. 4c, the navigation view 92 is displayed simultaneously. This allows navigating both instruments relative to the patient's body 18 at the same time by different surgeons operating on the different spatial zones.

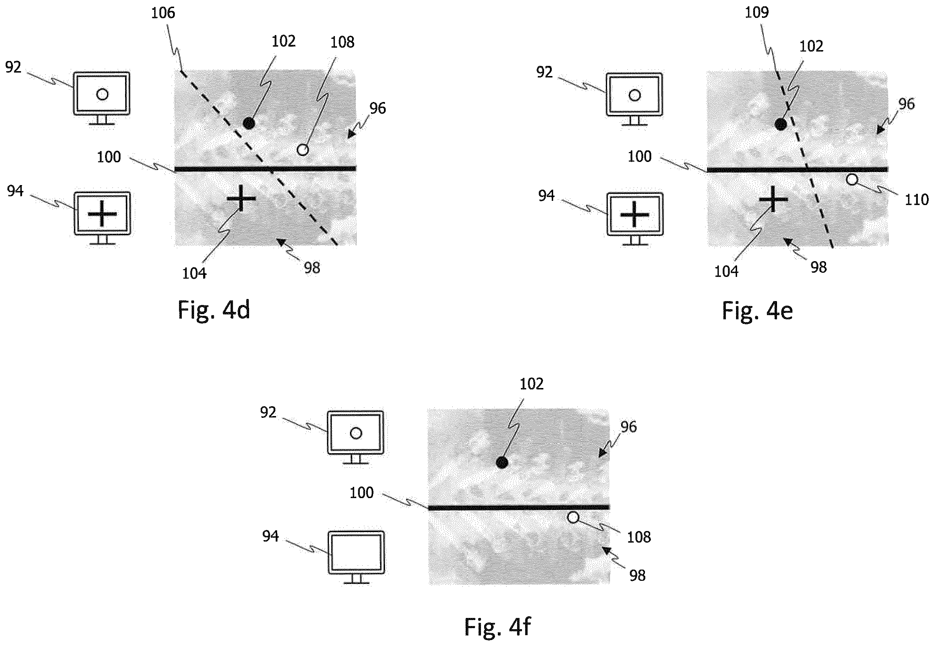

Fig. 4d illustrates a scenario in which the instrument associated with the spatial zone 96 is moved relative to the patient's body 18 from position 102 to position 108. Such a movement of the instrument may effect or trigger an update of the initial spatial region 96 and/or 98. In the illustrated example, the initial spatial zones 96, 98 are updated by using a virtual plane 106 instead of the virtual plane 100. The virtual plane 106 is perpendicular to a linear line connecting the positions 104 and 108 and intersects the linear line in its middle. This ensures that each of the positions 104, 108 has a similar distance to the edge of the respective spatial zone 96, 98. The association between the instruments and the updated spatial zones 96, 98 can be maintained and the navigation views can both be continually displayed in this scenario.

Fig. 4e illustrates a scenario in which the instrument associated with the spatial zone 96 is moved relative to the patient's body 18 from position 102 to position 110. Position 110 lies within the initial spatial zone 98 defined based on the virtual plane 100 as shown in Fig. 4a. To avoid the instrument from moving from between positions 102, 110 from the spatial zone 96 to the spatial zone 98, the initial spatial zones 96, 98 may be updated based on the instrument's movement. Again, the updated spatial zones 96, 98 may be defined by a virtual plane 109 perpendicular to a linear line connecting the positions 104 and 110 and intersecting that line in its middle. Also in this case, the association between the instruments and the spatial zones 96, 98 can be maintained and the navigation views 92, 94 can both be continually displayed.

In the scenario of Fig. 4f, the medical instrument previously located at position 104 is removed from the spatial region 98 and no longer within a tracking range of interest of the tracking system 6. Thus, the navigation view 94 may be left blank or stopped to be displayed. The navigation view 92 that is based on the instrument located at position 108 may not be affected by the removal of the instrument previously located at position 104. That is, the navigation view 92 may be continually displayed irrespective of a presence or absence of other medical instruments, as long as the medical instrument associated with the spatial zone 96 is within the tracking range of interest of the tracking system 6.

If, starting with the scenario of Fig. 4f, another medical instrument is positioned relative to the patient's body 18, that medical instrument may be associated with the (e.g., initial or updated) spatial region 98 if it is located within that region. This may lead to the same situation as shown in Fig. 4e. The initial spatial zones defined by the virtual plane 100 may be used as fixed zones used for associating a medical instrument to one of the spatial regions, and the spatial regions may subsequently be (e.g., continuously) updated based on (e.g., changed) tracked poses of the associated medical instruments.

Details of the inventive technique will now be phrased in other words to provide a better understanding thereof. Although reference signs of Figs. 1-4f may be used in the following, these are not to be understood as limiting the inventive technique to the specific details shown in these figures.

Complications (e.g. surgical site infections or blood loss) during a surgery may increase with the duration of the surgical intervention. More than one surgeon performing a navigated surgery simultaneously can decrease the surgical time and therefore the overall complication rate. Allowing navigation of multiple medical instruments 24, 26 simultaneously, so that each surgeon can operate on different vertebral levels or spinal sections at a time, can benefit in terms of reducing surgery time, reducing blood loss, and collaborating on a complex case. For example, in a scoliosis case where the spine 60 has a side-to-side bend which is estimated at 10 degrees or more, there may be a need for a spinal fusion. The basic motivation is to realign and fuse together some of the vertebrae 46-58 of the patient's spine 60 with additional support of rods, so that they heal into a single, solid bone. In such an operation, more than one surgeon may be operating on the patient's body 18 at a time. For example, one surgeon may implant pedicle screws on the patient's right side and another surgeon on the patient's left side of vertebral column. This may decrease turnaround time and reduce blood loss.

Some 3D navigation systems do not support simultaneous visualization of multiple navigated medical instruments. Only a view of a single medical instrument may be provided by such navigation systems. For example, in some systems, views are determined to be displayed (e.g., an axial view, a sagittal view, a coronal view, a view along a tool axis) using a tracked position of a distal tip 29 of the single medical instrument 26 as a reference for these views. If another medical instrument 24 is brought into a surgical region and is located closer to the patient's body 18 than the single instrument 26, then the single instrument 26 may no longer be considered as the primary medical instrument and the other medical instrument 24 may become the new primary medical instrument to be used for determining the view(s). In these cases, only one surgeon may navigate on the patient anatomy at a given time.

Some 2D navigation systems may visualize a view indicating multiple medical instruments 24, 26 at the same time. For example, a static view of a portion of the patient's body 18 (e.g., an anterior-posterior, AP, and a lateral x-ray image) is shown on a display unit. A navigated medical instrument 24 is displayed as an overlay on both x-ray images. In this case, multiple instruments 24, 26 can be overlaid in the x-ray images simultaneously. If the position of the medical instrument 24 effects a change in the view by changing a section of the x-ray images to be displayed as part of the view (e.g., in case the view corresponds to a zoomed version of an x-ray image close to the instrument's distal tip), a separate view for each navigated instrument 24, 26 may need to be provided. In 3D navigation systems, intersecting 2D planes of 3D patient image data may be shown on a display unit. The intersecting planes may be derived from the tracked position of the distal tip 29 of the medical instrument 26. In such a view, for example, a sagittal and/or a coronal intersecting plane may be shown which includes the instrument tip 29. The sagittal plane may be parallel to a y- axis and a z-axis, and the coronal plane may be parallel to an x-axis and a y-axis of a DICOM coordinate system of the 3D patient image data. In another view, also referred to as instrument-oriented view, the instrument tip 29 may be shown overlaid onto an intersecting plane, but the intersecting plane may in this case be defined by a coordinate system of the instrument tip 29 instead of the 3D patient image data. In this example, one could say that the instrument tip 29 scrolls through the 3D data set. In cases where the instrument tip 29 controls the displayed view, it may be desirable to display different views for each of a plurality of simultaneously used medical instruments 24, 26.

In navigated spinal surgery there might be a need to operate on several spinal levels or sections of a patient's spine 60 simultaneously by two or more surgeons. The technique disclosed herein allows providing simultaneous feedback for multiple tracked medical instruments 24, 26 associated with surgeon-specific spatial regions, for example by providing a separate navigation view 72, 74 for each of the instruments 24, 26. This may allow navigating multiple medical instruments 24, 26 simultaneously, so that each surgeon can operate on a different spinal level or section of the spine 60 of the patient's body 18 at a given time. For example, one surgeon may perform a spinal decompression on a right side of the patient's spine 60 while another can implant a pedicle screw on left side thereof, or the two surgeons may implant pedicle screws on the respective sides of the vertebral column of the patient's spine 60. The navigation views 72, 74 may be displayed on a single screen of a display unit 8 as illustrated in Fig. 1, or on individual screens thereof.

As another example for visual feedback, a blinking pattern and/or light intensity of an indicator lamp may indicate a distance and/or an alignment of the tracked medical instrument 26 relative to the associated spatial region 70. Alternatively, or in addition to providing a visual feedback such as a navigation view or a light signal of the indicator lamp as feedback on the tracked poses of the multiple medical instruments 24, 26, haptic and/or auditory feedback may be output to the user(s) in the operating room 22. For example, a vibration pattern and/or intensity of a haptic feedback unit worn by a surgeon may indicate a distance of the tracked medical instrument 24 relative to the associated spatial region 64. A warning tone may be output via a speaker to indicate that the distal tip 29 of the tracked medical instrument 26 is currently located outside the associated spatial region 70.

A surgeon may wish to sequentially use multiple tracked medical instruments during a procedure. The medical instrument that is actively used by a surgeon for surgical navigation and shall be used for providing feedback to the surgeon may be referred to as primary instrument 24, 26. The presented technique allows providing feedback for multiple primary medical instruments 24, 26 handled simultaneously, for example by displaying navigation views 72, 74 for the multiple primary medical instruments 24, 26 while other medical instruments may or may not be visualized in these navigation views.

A primary medical instrument 24, 26 may be selected from a plurality of tracked medical instruments by a user, for example by pressing a button on the primary medical instrument 24, 26 or on a Graphical User Interface. Alternatively, a primary medical instrument 24, 26 may be determined based on the tracking data and the spatial data, for example in case the tracked pose of the primary instrument 24, 26 fulfils the one or more predefined spatial constraints. In one particular example, a medical instrument 26 can be selected as primary medical instrument if its distal tip 29 is located in the associated spatial region 70. If more than one medical instrument lies in a same spatial region 62-70, other criteria may be used for selecting the primary instrument from the more than one medical instrument, for example a tool located closest to the patient tracker 33 or closest to a center of the same spatial region 62-70 may be selected as the primary medical instrument. As another option, each medical instrument may have an associated priority that may be predefined (e.g., based on a type of the medical instrument) or based on the tracking information (e.g., based on a movement direction or movement speed of the medical instrument), and the primary instrument may be selected from multiple tracked medical instruments based on the priorities of these medical instruments.

The present technique may enable simultaneous navigation of multiple medical instruments 24, 26, for example by providing corresponding navigation views 72, 74 to surgeons. One concept lies in providing a plurality of spatial regions 62-70. One primary medical instrument may be associated to each spatial region 62-70 or to a subset of the spatial regions 62-70. The patient's body 18 may be divided into multiple surgical zones as spatial regions 62-70 that are operated by different surgeons, for example by using separating virtual planes 65, 67, 78, 68, 88, 90 defining the spatial regions 62-70. The spatial regions 62-70 may be defined based on one or more of the following approaches (A) to (D).

### (a) virtual Plane based on anatomical plane

A sagittal plane derived from the coordinate system of the patient image data (e.g., a DICOM image dataset) may be used as a virtual plane to divide the patient's body 18 into a left and a right region. Several planes could be combined to create more spatial regions. For example, an additional transverse plane may be added to divide the left and right regions into cranial and a caudal sub-regions, resulting in a total of four spatial regions. Instead of using the anatomical planes as the virtual planes for defining the spatial regions, one may use planes that are parallel to the anatomical planes and which, for example, contain a (e.g., predefined) point of interest. Examples of such virtual planes 86, 88, 90 corresponding to or parallel to the anatomical planes are shown in Fig. 3d.

### (b) virtual plane based on anatomical element(s)

The orientation of a virtual plane does not necessarily need to correspond to an anatomical axis as defined by the coordinate system of the patient image data. In another variant, the orientation of a virtual plane is defined relative to an anatomical element of the patient's body 18, such as a bone (e.g., a vertebra 46-58), an organ or a tumor. The orientation of the virtual plane may be derived from a segmentation of this anatomical element. One or more of the virtual planes may be determined based on a segmentation of the patient's spine 60. As an example, a virtual plane may approximate an endplate of a vertebra of the patient's body, as exemplarily illustrated in Fig. 3a. In this case, one surgeon may operate on superior levels relative to this virtual plane and another surgeon may operate on inferior levels in respect to this virtual plane. Several virtual planes could be defined in a similar manner, thereby dividing the patient's spine 60 into different spatial regions. Another example of a virtual plane defined based on an anatomical element of the patient's body is a virtual plane that approximates the spinous process of a vertebra and divides the patient's body 18 into a left and a right side, as exemplarily illustrated in Fig. 3b.

### (c) virtual plane defined by a user

A user may define a spatial region and/or one or more of the virtual planes. For example, the one or more virtual planes can be defined by the user via a Graphical User Interface, GUI, based on the patient image data. In one example, the user defines two points in the patient image data to define an axis, and defines a point on this axis. A virtual plane may then be determined having the user-defined axis as normal and the user-defined point as intersection point with the user-defined axis. As another example, the user may draw freeform shapes in a plurality of views of the patient's body 18 as represented by the patient image data, and a spatial region may be determined which has the respective freeform shapes as contour. Other variants for defining virtual planes or spatial regions by a user are also possible.

### (d) General geometric object

The discriminating geometric object used to define a spatial region is not restricted to be a virtual plane. A general geometric (e.g., virtual) object such as a mesh-based surface model may be used to define one or more of the spatial regions. For example, an anatomical element in the patient image data may be segmented and a convex hull may be defined around the segmented anatomical element as a spatial region, as exemplarily illustrated in Fig. 3d. This spatial region may thus have a sub volume of the patient's body 18 as an interior or inside volume, and may divide the patient's body 18, and the patient image data representative of the patient's body 18, into an inside volume and outside volume.

In all cases (A) to (D), spatial regions can further be restricted by a bounding volume, for example a bounding box around the patient's body 18 and/or the patient image data.

When dividing the anatomy into *n* different spatial regions, the number of primary medical instruments may range from zero to *n.* A medical instrument 26, the distal tip 29 of which lies in one of the defined spatial regions, may be associated with this one of the spatial regions and thus become the primary medical instrument for this associated one of the spatial regions.

A navigation view 74 may be provided as visual feedback related to this primary medical instrument 26, the navigation view indicating a pose of the primary instrument 26 relative to the portion of the patient's body 18 as represented by the patient image data, the portion being contained in the spatial region 70 associated to the primary instrument 24. Each spatial region 62-70 can be navigated simultaneously and independently using a respective primary instrument 24, 26. During navigation, spatial regions 64, 70 associated with the primary instruments 24, 26 may be visualized concurrently on a single screen (e.g., as illustrated in Fig. 1) or on multiple screens. Multiple navigation views 72, 74 can be displayed on different windows of a same display unit 8 or on different display units. Each primary instrument 24, 26 may be visualized in its own navigation view(s) 72, 74. In an anatomical navigation view this may for example be a 2D sagittal and 2D coronal view which contains the tip of the primary instrument. This may enable surgeons to collaborate and operate together.

Each of the tracked medical instruments may be classified (e.g., by the tracking system) into a surgeon-specific set of medical instruments. Different surgeons may use different instrument sets. These sets may be predefined or defined by a user (e.g., the surgeon(s)). In one example, one instrument per instrument set is used as a primary instrument. This means that only one instrument per instrument set may be associated to a spatial region 62-70 or a subset of spatial regions 62-70, and feedback may be triggered using the one or more feedback parameters of the spatial region(s) 62-70 associated with that primary instrument.

As explained above, one concept lies in dividing the patient's anatomy into two or more spatial regions 62-70 and associating one primary navigated medical instrument 24, 26 to a spatial region. A fixed association between a medical instrument 24, 26 and a spatial region 62-70 can be used. The displayed navigation view indicating a pose of the associated medical instrument 24, 26 may be determined for the respective associated spatial region 62-70 to provide each surgeon with a dedicated visualization (e.g., on a surgeon-specific display unit). In contrast to such a region-based approach for identifying which portion of the patient's body to display in the navigation view, a direct association of a medical instrument to a display unit would not make it possible to visualize the same medical instrument on different display units for the surgeons without processing additional information like tracking of the surgeons to identify which instrument belongs to whom or adjusting manually for each display unit which tool should be used for navigation.

If a distal tip of a medical instrument of a surgeon A enters a spatial region defined for another surgeon B, there may no longer be a primary medical instrument in the spatial region for surgeon A, but two medical instruments may be simultaneously located in the spatial region for surgeon B. Only one medical instrument may be the primary one that controls the navigation view. Thus, in this scenario, one surgeon might "loose" his navigation view. For example, surgeon B may no longer be provided with a navigation view. This means A is taking over the control, where B does not see his instrument visualized anymore. To avoid this situation, the spatial regions may be dynamically updated once multiple surgeons are navigating simultaneously. The segregation between the spatial regions may be based on the current position of the tips of the tracked medical instruments. That is, static regions (e.g., defined relative to the patient's body) may be used initially and be updated subsequently based on a movement of the tracked medical instruments, in particular to avoid a tracked medical instrument from moving into another spatial region. One may say that the static regions can be employed as triggers to visualize a medical instrument on a specific display unit for a surgeon.

Once multiple surgeons are navigating the tracked medical instruments simultaneously and the navigation views are displayed (e.g., on dedicated display units for each surgeon), the respective associated spatial regions may be anchored to the distal tips of the tracked medical instruments. In other words, the segregation of the situs may be derived by the positions of the medical instruments.

For example, a virtual plane separating two adjacent spatial regions from one another may be a defined as a plane that is perpendicular to a line connecting the distal tips of two tracked (e.g., primary) medical instruments, which plane has the same distance to both distal tips. This concept of dividing the space into spatial regions is known from Voronoi diagrams. That is, each of the (e.g., updated) plurality of spatial regions may correspond to a Voronoi cell defined based on a point (e.g., a distal tip) of a tracked medical instrument. In a Voronoi diagram for a given set of points {*p₁, ..., pₙ*} the space is divided into so called Voronoi cells. The Voronoi cell for the point *pᵢ* consists of every point whose distance to *pᵢ* is less than its distance to any other point of the given point set. So, for *n* surgeons, *n* segregating virtual planes may be defined, thereby defining *n* Voronoi cells, one for each of the instrument tips *pᵢ, i* = 1, ..., *n.* With this concept of dynamically dividing the space into Voronoi cells for each instrument tip, it is possible to provide a fixed navigation view for each surgeon, even if the tip of the primary instrument associated to that surgeon enters a spatial region that was initially provided for another surgeon.

If multiple surgeons are operating and one surgeon removes a medical instrument from the situs, the associated navigation view may be let blank while the others still show their navigated instrument tips. If the surgeon picks another medical instrument and inserts the instrument's tip into the spatial region defined for that surgeon, this instrument may again be visualized in the navigation view previously left blank. If there is already at least one instrument in that spatial region, a separation (e.g., a subdivision of that spatial region) may be performed with the new instrument and other instrument(s) in said region. The new instrument's tip may be shown on the display unit associated to the surgeon and all other instruments may still be visualized in their navigation view and/or on their display units.

Various modifications of the technique disclosed herein are possible. For example, technique presented herein is not restricted to spinal surgery and applies to other navigated surgical procedures (e.g., hip, knee, cranial or shoulder surgery).

The technique may allow more than one surgeon to navigate his primary medical instrument 24, 26 simultaneously during a surgical navigation procedure. Navigating with multiple medical instruments 24, 26 at the same time decreases the intervention duration and increases efficiency and effectivity of the intervention. When providing the feedback based on the spatial region associated with a tracked instrument, instead of providing a same feedback for all tracked instruments, a user may distinguish the feedback between the spatial regions 62-70, thereby correlating the respective primary instrument with the associated spatial region more easily. Region-specific feedback parameters may enable a feedback that is specifically tailored to the respective spatial region, which may be particularly advantageous compared with solutions in which the feedback on a tracked instrument pose is the same irrespective of the instrument's pose relative to a spatial region. When the spatial regions are associated to different surgeons, the one or more feedback parameters may be tailored to the needs and preferences of the different surgeons. Further advantages may become apparent to those skilled in the art in view of the present disclosure.

## Claims

1. A computer-implemented method for supporting users in an operating room (22) by triggering feedback regarding one or more medical instruments (24; 26), the method being performed by at least one processor (12) and comprising:
obtaining (202) spatial information indicative of a plurality of spatial regions (62-70) in the operating room (22), each of the plurality of spatial regions (62-70) being associated with one or more feedback parameters;
obtaining (204) tracking information indicative of tracked poses of a plurality of medical instruments (24; 26) in the operating room (22);
obtaining one or more predefined spatial constraints for one or more of the plurality of spatial regions;
determining, based on the spatial information and the tracking information, whether the one or more predefined spatial constraints are met;
associating (206), based on the spatial information and the tracking information, each of the plurality of medical instruments (24; 26) to at least one of the plurality of spatial regions (62-70) based on whether the one or more predefined spatial constraints of said at least one of the plurality of spatial regions are met; and
triggering (208) feedback, for each of the medical instruments (24, 26), according to the one or more feedback parameters of the associated at least one of the plurality of spatial regions (62-70), wherein the one of more feedback parameters define a visual feedback that includes display of a navigation view (72, 74) visualizing the pose of the respective medical instrument (24, 26) and wherein the one or more feedback parameters define at least one setting of the navigation view (72, 74), the at least one setting comprising:
a type of medical image data used for rendering the navigation view (72, 74);
a criterion for highlighting structures in the navigation view (72, 74);
a criterion for indicating planned objects in the navigation view (72, 74);
an orientation of the navigation view (72, 74); and/or
a perspective of the navigation view (72, 74).

2. The method of claim 1, wherein the one or more predefined spatial constraints comprise at least one of:
a maximum distance between the instrument and the spatial region,
a maximum distance between the instrument and an anatomical element enclosed by and/or defining the spatial region,
a maximum deviation of a main instrument axis from the spatial region, and
a maximum deviation of a main instrument axis from an anatomical element enclosed by and/or defining the spatial region.

3. The method of claim 1 or 2, wherein at least one of the spatial information and the tracking information is obtained for multiple points in time and the feedback is iteratively triggered for two or more of the multiple points in time.

4. The method of any one of claims 1 to 3, wherein the plurality of spatial regions are separated from one another by one or more virtual planes defined relative to a patient's body.

5. The method of any one of claims 1 to 4, wherein the spatial information is determined or defined based on patient image data comprising one or more medical images of at least a portion of a patient's body.

6. The method of any one of claims 1 to 5, wherein
the one or more feedback parameters are region-specific and/or differ between two or more of the plurality of spatial regions (62-70).

7. The method of any one of claims 1 to 6, wherein
the plurality of spatial regions (62-70) are defined based on a pose of at least one reference object in the operating room (22).

8. The method of claim 7, wherein
the at least one reference object (22) comprises at least a portion of a patient's body (18) and/or at least a portion of a medical tool (28) and/or at least a portion of a medical instrument (24, 26).

9. The method of claim 8, wherein
different ones of the plurality of spatial regions (62-70) comprise different portions of the patient's body (18).

10. The method of claim 9, wherein
at least some of the plurality of spatial regions (62-70) are separated from one another by one or more virtual planes (65, 67, 78, 68, 88, 90) that are optionally defined relative to the patient's body (18); wherein, optionally, the one or more virtual planes (65, 67, 78, 86, 88, 90) comprise at least one anatomical plane, at least one user-defined plane and/or at least one plane associated with an anatomical element of the patient's body (18).

11. The method of claim 3, wherein
one or more of the associated at least one of the plurality of spatial regions are updated based on a movement of the medical instrument as indicated by the tracking information obtained for multiple points in time; wherein, optionally:
the associated at least one of the plurality of spatial regions is updated such that the medical instrument remains within said at least one of the plurality of spatial regions, and/or
the associated at least one of the plurality of spatial regions is updated such that another medical instrument not associated with said at least one of the plurality of spatial regions remains outside said at least one of the plurality of spatial regions.

12. The method of any one of claims 1 to 11, wherein different subsets of the plurality of spatial regions (65, 67, 78, 86, 88, 90) are associated with different surgeons.

13. A system (2) comprising at least one processor (12) configured to perform the method according to any one of claims 1 to 12.

14. The system (2) of claim 13, further comprising at least one of the following components:
a tracking system (6) configured to track the pose of the medical instrument (24; 26) in the operating room (22);
a feedback unit (8) configured to provide the feedback to at least one user in the operating room (22);
a robot (10) configured to handle a medical tool (28).

15. A computer program comprising instructions which, when executed on at least one processor (12), cause the at least one processor (12) to perform the method according to any one of claims 1 to 12, the computer program being optionally carried by a carrier (14).

## Patentansprüche

1. Computerimplementiertes Verfahren zur Unterstützung von Benutzern in einem Operationssaal (22) durch Auslösen einer Rückmeldung bezüglich eines oder mehrerer medizinscher Instrumente (24; 26), wobei das Verfahren durch mindestens einen Prozessor (12) durchgeführt wird und umfasst:
Erfassen (202) räumlicher Informationen, die eine Vielzahl räumlicher Regionen (62-70) im Operationssaal (22) indizieren, wobei jede der Vielzahl räumlicher Regionen (62-70) mit einem oder mehreren Rückmeldungsparametern assoziiert ist;
Erfassen (204) Verfolgungsinformationen, die verfolgte Posen einer Vielzahl medizinischer Instrumente (24; 26) in einem Operationssaal (22) indizieren;
Erfassen einer oder mehrerer vordefinierten räumlichen Bedingungen für eine oder mehrere der Vielzahl räumlicher Regionen;
Bestimmen, basierend auf den räumlichen Informationen und den Verfolgungsinformationen, ob die eine oder mehreren vordefinierten räumlichen Bedingungen eingehalten werden;
Assoziieren (206), basierend auf den räumlichen Informationen und den Verfolgungsinformationen, jedes der Vielzahl medizinischer Instrumente (24; 26) zu mindestens einer der Vielzahl räumlicher Regionen (62-70) basierend darauf, ob die eine oder mehreren vordefinierten räumlichen Bedingungen der genannten mindestens einen der Vielzahl räumlicher Regionen eingehalten werden; und
Auslösen (208) einer Rückmeldung, für jedes der medizinischen Instrumente (24,26), entsprechend dem einen oder den mehreren Rückmeldungsparametern der assoziierten mindestens einen der Vielzahl räumlicher Regionen (62-70), wobei der eine oder die mehreren Rückmeldungsparameter eine visuelle Rückmeldung definieren, die ein Anzeigen einer Navigationsansicht (72, 74) beinhaltet, die die Pose des jeweiligen medizinischen Instruments (24, 26) darstellt und wobei der eine oder die mehreren Rückmeldungsparameter mindestens eine Einstellung der Navigationsansicht (72, 74) definieren, die mindestens eine Einstellung umfassend:
eine Art von medizinischen Bilddaten, die zur Darstellung der Navigationsansicht (72, 74) genutzt werden;
ein Kriterium zur Hervorhebung von Strukturen in der Navigationsansicht (72, 74);
ein Kriterium zur Assoziierung von geplanten Objekten in der Navigationsansicht (72, 74);
eine Ausrichtung der Navigationsansicht (72, 74); und/oder
eine Perspektive der Navigationsansicht (72, 74).

2. Verfahren nach Anspruch 1, wobei die eine oder mehreren vordefinierten räumlichen Bedingungen umfassen mindestens eines aus:
einem maximalen Abstand zwischen dem Instrument und der räumlichen Region,
einem maximalen Abstand zwischen dem Instrument und einem anatomischen Element, das die räumliche Region umschließt und/oder definiert,
einer maximalen Abweichung einer Hauptinstrumentenachse von der räumlichen Region, und
einer maximalen Abweichung einer Hauptinstrumentenachse von einem anatomischen Element, das die räumliche Region umschließt und/oder definiert.

3. Verfahren nach Anspruch 1 oder 2, wobei mindestens eine aus den räumlichen Informationen und den Verfolgungsinformationen für zahlreiche Zeitpunkte erfasst wird und die Rückmeldung für zwei oder mehr der zahlreichen Zeitpunkte iterativ ausgelöst wird.

4. Verfahren nach einem der Ansprüche 1 bis 3, wobei die Vielzahl räumlicher Regionen durch eine oder mehrere virtuellen Ebenen voneinander getrennt sind, die relativ zum Körper eines Patienten definiert sind.

5. Verfahren nach einem der Ansprüche 1 bis 4, wobei die räumlichen Informationen basierend auf den Patientenbilderdaten bestimmt oder definiert werden, die ein oder mehrere medizinische Bilder von mindestens einem Teil des Körpers eines Patienten umfassen.

6. Verfahren nach einem der Ansprüche 1 bis 5, wobei
der eine oder die mehreren Rückmeldungsparameter regionenspezifisch sind und/oder sich zwischen zwei oder mehreren der Vielzahl räumlicher Regionen (62-70) unterscheiden.

7. Verfahren nach einem der Ansprüche 1 bis 6, wobei
die Vielzahl räumlicher Regionen (62-70) basierend auf einer Pose mindestens eines Referenzobjekts in dem Operationsraum (22) definiert sind.

8. Verfahren nach Anspruch 7, wobei
das mindestens eine Referenzobjekt (22) mindestens einen Teil des Körpers eines Patienten (18) und/oder mindestens einen Teil eines medizinischen Werkzeugs (28) und/oder mindestens einen Teil eines medizinischen Instruments (24, 26) umfasst.

9. Verfahren nach Anspruch 8, wobei
verschiedene der Vielzahl räumlicher Regionen (62-70) verschiedene Teile des Körpers des Patienten umfassen.

10. Verfahren nach Anspruch 9, wobei
mindestens einige der Vielzahl räumlicher Regionen (62-70) durch eine oder mehrere virtuellen Ebenen (65, 67, 78, 68, 88, 90) voneinander getrennt sind, die optional relativ zum Körper des Patienten (18) definiert sind; wobei, optional, die eine oder mehreren Ebenen (65, 67, 78, 68, 88, 90) umfassen mindestens eine anatomische Ebene, mindestens eine benutzerdefinierte Ebene und/oder mindestens eine Ebene, die einem anatomischen Element des Körpers des Patienten (18) assoziiert ist.

11. Verfahren nach Anspruch 3, wobei
eine oder mehrere der assoziierten mindestens eine der Vielzahl räumlicher Regionen basierend auf einer Bewegung des medizinischen Instruments aktualisiert werden, wie sie durch die für zahlreiche Zeitpunkte erfassten Verfolgungsinformationen indiziert werden; wobei, optional:
die assoziierten mindestens eine der Vielzahl räumlicher Regionen derart aktualisiert wird, dass das medizinische Instrument innerhalb der genannten mindestens eine der Vielzahl räumlicher Regionen verbleibt, und/oder
die assoziierte mindestens eine aus der Vielzahl räumlicher Regionen derart aktualisiert wird, dass ein anderes medizinischen Instrument, das nicht mit der genannten mindestens einer der Vielzahl räumlicher Regionen assoziiert ist, außerhalb der genannten mindestens einer der Vielzahl räumlicher Regionen verbleibt.

12. Verfahren nach einem der Ansprüche 1 bis 11, wobei verschiedene Teilmengen der Vielzahl räumlicher Regionen (65, 67, 78, 86, 88, 90) verschiedenen Chirurgen assoziiert sind.

13. System (2) umfassend mindestens einem Prozessor (12), dazu konfiguriert, das Verfahren entsprechend nach einem der Ansprüche 1 bis 12 durchzuführen.

14. System (2) nach Anspruch 13, ferner umfassend mindestens eines aus den folgenden Komponenten:
ein Verfolgungssystem (6), dazu konfiguriert, die Pose des medizinischen Instruments (24; 26) im Operationssaal (22) zu verfolgen;
eine Rückmeldungseinheit (8), dazu konfiguriert, die Rückmeldung an mindestens einen Benutzer in dem Operationssaal (22) zu übermitteln;
einen Roboter (10), dazu konfiguriert, ein medizinisches Werkzeug (28) zu bedienen.

15. Computerprogramm umfassend Anweisungen, welche, wenn mindestens auf einem Prozessor (12) ausgeführt, den mindestens einen Prozessor (12) veranlassen das Verfahren entsprechend nach einem der Ansprüche 1 bis 12 durchzuführen, wobei das Computerprogramm optional auf einem Datenträger (14) gespeichert ist.

## Revendications

1. Procédé mis en œuvre par ordinateur destiné à aider les utilisateurs dans une salle d'opération (22) en générant des informations de retour concernant un ou plusieurs instruments médicaux (24; 26), le procédé étant exécuté par au moins un processeur (12) et comprenant:
l'obtention (202) d'informations spatiales indiquant une pluralité de zones spatiales (62-70) dans la salle d'opération (22), chaque zone de la pluralité des zones spatiales (62-70) étant associée à un ou plusieurs paramètres d'informations de retour;
l'acquisition (204) d'informations de suivi indiquant les positions suivies d'une pluralité d'instruments médicaux (24 ; 26) dans la salle d'opération (22);
l'obtention d'une ou plusieurs contraintes spatiales prédéfinies pour une ou plusieurs des différentes zones spatiales;
la détermination, à partir des informations spatiales et des informations de suivi, si la ou les contraintes spatiales prédéfinies sont respectées;
l'association (206), sur la base des informations spatiales et des informations de suivi, de chaque instrument médical de la pluralité des instruments médicaux (24; 26) à au moins l'une parmi la pluralité des zones spatiales (62-70), selon que la ou les contraintes spatiales prédéfinies de ladite au moins une zone de la pluralité des zones spatiales sont respectées; et
le déclenchement (208) d'informations de retour, pour chacun des instruments médicaux (24, 26), en fonction d'un ou plusieurs paramètres d'informations de retour de l'au moins une zone associée parmi la pluralité de zones spatiales (62-70), dans lequel le ou les paramètres d'informations de retour définissent une information de retour visuelle qui comprend l'affichage d'une vue de navigation (72, 74) visualisant la pose de l'instrument médical respectif (24, 26) et dans lequel le ou les paramètres d'informations de retour définissent au moins un réglage de la vue de navigation (72, 74), l'au moins un réglage comprenant:
un type de données d'image médicale utilisée pour la restitution de la vue de navigation (72, 74);
un critère permettant de mettre en évidence des structures dans la vue de navigation (72, 74);
un critère permettant d'indiquer les objets planifiés dans la vue de navigation (72, 74);
une orientation de la vue de navigation (72, 74); et/ou
une vue en perspective de la vue de navigation (72, 74).

2. Procédé selon la revendication 1, dans lequel la ou les contraintes spatiales prédéfinies comprennent au moins l'une parmi:
une distance maximale entre l'instrument et la zone spatiale,
une distance maximale entre l'instrument et un élément anatomique entouré par la zone spatiale et/ou délimitant cette dernière,
un écart maximal d'un axe principal de l'instrument par rapport à la zone spatiale, et
un écart maximal d'un axe principal de l'instrument par rapport à un élément anatomique entouré par la zone spatiale et/ou délimitant cette dernière.

3. Procédé selon la revendication 1 ou 2, dans lequel au moins l'une parmi les informations spatiales et les informations de suivi est obtenue pour plusieurs points temporels et les informations de retour sont déclenchées de manière itérative pour au moins deux des multiples points temporels.

4. Procédé selon l'une quelconque des revendications 1 à 3, dans lequel la pluralité de zones spatiales sont séparées les unes des autres par un ou plusieurs plans virtuels définis par rapport au corps d'un patient.

5. Procédé selon l'une quelconque des revendications 1 à 4, dans lequel les informations spatiales sont déterminées ou définies sur la base de données images du patient comprenant une ou plusieurs images médicales d'au moins une partie du corps d'un patient.

6. Procédé selon l'une quelconque des revendications 1 à 5, dans lequel
le ou les paramètres d'informations de retour sont spécifiques à une zone à une zone et/ou diffèrent entre deux ou plusieurs de la pluralité de zones spatiales (62-70).

7. Procédé selon l'une quelconque des revendications à 6, dans lequel
la pluralité de zones spatiales (62-70) sont définies en fonction de la position d'au moins un objet de référence dans la salle d'opération (22).

8. Procédé selon la revendication 7, dans lequel
l'au moins un objet de référence (22) comprend au moins une partie du corps d'un patient (18) et/ou au moins une partie d'un outil médical (28) et/ou au moins une partie d'un instrument médical (24, 26).

9. Procédé selon la revendication 8, dans lequel
certaines parmi la pluralité des zones spatiales (62-70) correspondent à différentes parties du corps du patient (18).

10. Procédé selon la revendication 9, dans lequel
au moins certaines parmi la pluralité des zones spatiales (62 à 70) sont séparées les unes des autres par un ou plusieurs plans virtuels (65, 67, 78, 68, 88, 90) qui sont, éventuellement, définis par rapport au corps du patient (18); dans lequel, éventuellement, le ou les plans virtuels (65, 67, 78, 86, 88, 90) comprennent au moins un plan anatomique, au moins un plan défini par l'utilisateur et/ou au moins un plan associé à un élément anatomique du corps du patient (18).

11. Procédé selon la revendication 3, dans lequel
une ou plusieurs de l'au moins une zone associée de la pluralité des zones spatiales sont mises à jour en fonction d'un mouvement de l'instrument médical indiqué par les informations de suivi obtenues à de multiples points temporels; dans lequel, éventuellement:
l'au moins une zone associée de la pluralité des zones spatiales est mise à jour de manière à ce que l'instrument médical reste à l'intérieur de ladite au moins une zone parmi ladite pluralité de zones spatiales, et/ou
l'au moins une zone associée parmi la pluralité des zones spatiales sont mises à jour de telle sorte qu'un autre instrument médical non associé à ladite au moins une zone parmi la pluralité de zones spatiales reste en dehors de ladite au moins une zone parmi la pluralité des zones spatiales.

12. Procédé selon l'une quelconque des revendications 1 à 11, dans lequel différents sous-ensembles de la pluralité de zones spatiales (65, 67, 78, 86, 88, 90) sont associés à différents chirurgiens.

13. Système (2) comprenant au moins un processeur (12) configuré pour mettre en œuvre le procédé selon l'une quelconque des revendications 1 à 12.

14. Système (2) selon la revendication 13, comprenant en outre au moins l'un des composants suivants:
un système de suivi (6) configuré pour suivre la position de l'instrument médical (24; 26) dans la salle d'opération (22);
une unité d'informations de retour (8) conçue pour fournir les informations de retour à au moins un utilisateur présent dans la salle d'opération (22);
un robot (10) conçu pour manipuler un instrument médical (28).

15. Programme informatique comprenant des instructions qui, lorsqu'elles sont exécutées sur au moins un processeur (12), amènent l'au moins un processeur (12) à mettre en œuvre le procédé selon l'une quelconque des revendications 1 à 12, le programme informatique étant éventuellement supporté par un support (14).
